(19) Europäisches Patentamt
European Patent Office
Office européen des brevets

(11) Publication number: 0 548 798 A1

(12) EUROPEAN PATENT APPLICATION

(21) Application number: 92121466.4

(22) Date of filing: 17.12.92

(51) Int. Cl.5: C07D 295/18, C07D 295/22, C07D 295/06, C07D 403/06, C07D 401/04, C07D 403/04, C07D 405/12, C07D 471/04, C07D 473/30, A61K 31/495

(30) Priority: 18.12.91 JP 335028/91

(43) Date of publication of application: 30.06.93 Bulletin 93/26

(84) Designated Contracting States: AT BE CH DE DK ES FR GB GR IE IT LI LU MC NL PT SE

(71) Applicant: SANWA KAGAKU KENKYUSHO CO., LTD.
No. 35, Higashi-sotobori-cho
Higashi-ku Nagoya-shi Aichi-ken(JP)

(72) Inventor: Kurono, Masayasu, c/o Sanwa Kagaku Kenkyusho Co.
Ltd., 35, Higashisotobori-cho
Higashi-ku, Nagoya-shi, Aichi(JP)
Inventor: Baba, Yutaka, c/o Sanwa Kagaku Kenkyusho Co., Ltd.
35, Higashisotobori-cho
Higashi-ku, Nagoya-shi, Aichi(JP)
Inventor: Iwata, Noriyuki, c/o Sanwa Kagaku Kenkyusho Co.
Ltd., 35, Higashisotobori-cho
Higashi-ku, Nagoya-shi, Aichi(JP)
Inventor: Kakigami, Takuji, c/o Sanwa Kagaku Kenkyusho Co.
Ltd., 35, Higashisotobori-cho
Higashi-ku, Nagoya-shi, Aichi(JP)
Inventor: Isogawa, Kogaku, c/o Sanwa Kagaku Kenkyusho Co.
Ltd., 35, Higashisotobori-cho
Higashi-ku, Nagoya-shi, Aichi(JP)
Inventor: Mitani, Takahiko, c/o Sanwa Kagaku Kenkyusho Co.
Ltd., 35, Higashisotobori-cho
Higashi-ku, Nagoya-shi, Aichi(JP)
Inventor: Ishiwata, Yoshiro, c/o Sanwa Kagaku Kenkyusho Co.
Ltd., 35, Higashisotobori-cho
Higashi-ku, Nagoya-shi, Aichi(JP)
Inventor: Yokochi, Shoji, c/o Sanwa Kagaku Kenkyusho Co.
Ltd., 35, Higashisotobori-cho
Higashi-ku, Nagoya-shi, Aichi(JP)
Inventor: Otsuka, Tamaki, c/o Sanwa Kagaku Kenkyusho Co.
Ltd., 35, Higashisotobori-cho
Higashi-ku, Nagoya-shi, Aichi(JP)
Inventor: Asano, Kyoichi, c/o Sanwa Kagaku Kenkyusho Co.
Ltd., 35, Higashisotobori-cho
Higashi-ku, Nagoya-shi, Aichi(JP)
Inventor: Ohwaki, Hiroyuki, c/o Sanwa Kagaku Kenkyusho Co.
Ltd., 35, Higashisotobori-cho
Higashi-ku, Nagoya-shi, Aichi(JP)
Inventor: Sawai, Kiichi, c/o Sanwa Kagaku Kenkyusho Co.
Ltd., 35, Higashisotobori-cho
Higashi-ku, Nagoya-shi, Aichi(JP)

(74) Representative: Kraus, Walter, Dr. et al
Patentanwälte Kraus, Weisert & Partner
Thomas-Wimmer-Ring 15
W-8000 München 22 (DE)

(54) **Antiviral agent.**

(57) An antiviral agent containing a compound represented by formula (I):

EP 0 548 798 A1

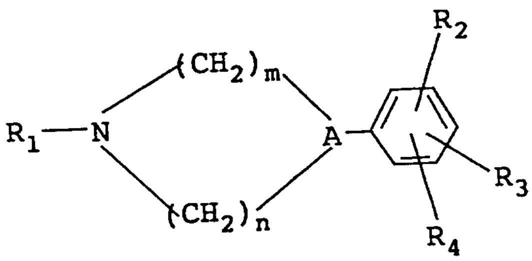

(I)

wherein $R_1$ represents a substituted or unsubstituted alkyl group, a substituted or unsubstituted acyl group, a substituted or unsubstituted arylsulfonyl group, a substituted or unsubstituted alkylsulfonyl group, a substituted or unsubstituted phenyl group, or a substituted or unsubstituted heterocyclic group; $R_2$, $R_3$, and $R_4$, which may be the same or different, each represent a hydrogen atom, an amino group, a substituted or unsubstituted alkylamino group, an acylamino group, a substituted or unsubstituted alkyl group, a hydroxyl group, a substituted or unsubstituted alkyloxy group, a halogen atom, a carboxyl group, a substituted or unsubstituted alkylcarbonyl group, a substituted or unsubstituted alkoxycarbonyl group, a substituted or unsubstituted aryloxycarbonyl group, a substituted or unsubstituted carbamoyl group, a nitro group, a cyano group, a thiol group, a substituted or unsubstituted alkylthio group, a substituted or unsubstituted phenyl group, or a substituted or unsubstituted heterocyclic group; A represents a nitrogen group or a methylene group; m represents 0 or a natural number; and n represents a natural number,
or a pharmaceutically acceptable salt thereof, are disclosed.

## FIELD OF THE INVENTION

This invention relates to a prophylactic and treating agent for infectious diseases caused by various DNA viruses, RNA viruses, and retroviruses.

## BACKGROUND OF THE INVENTION

Substituted or unsubstituted phenylpiperazine, phenylpiperidine, phenylpyrrolidine, phenyltetrahydroimidazole, and phenylhomopiperazine have hitherto been utilized as a useful substituent group in the production of a number of pharmaceutical compounds. However, antiviral compounds having these compounds per se as a basic skeleton or a center of activity are unknown.

There are only a few known antiviral compounds containing these compounds as a substituent, for example, glycyrrhetic acid derivatives discovered by the same inventors of the present invention (see Japanese Patent Application No. 127271/91), the compounds discovered by the same inventors of the present invention and disclosed in Japanese Patent Application No. 306597/91, pyridazine derivatives (see U.S. Patent 5,001,125, European Patent Publication No. 156433A and JP-A-60-226862 (the term "JP-A" as used herein means an "unexamined published Japanese patent application")), and ketoconazole (see Antimicrobial Agents and Chemotherapy, Vol. 21 (2), pp. 358-361 (1982)).

Of these known compounds having a substituted or unsubstituted phenylpiperazine, phenylpiperidine, phenylpyrrolidine, phenyltetrahydroimidazole or phenylhomopiperazine group, those having been proved to have an anti-herpesvirus activity are limited to the glycyrrhetic acid derivatives (Japanese Patent Application No. 127271/91), the compounds of Japanese Patent Application No. 306597/91, and ketoconazole (Antimicrobial Agents and Chemotherapy, Vol. 30 (2), pp. 215-219 (1986)). The above-mentioned pyridazine derivatives of European Patent Publication EP 156433A are known to be effective on viruses belonging to Picornaviridae typically including Rhinovirus (common cold virus) but unknown with respect to antiviral activity on viruses belonging to Herpesviridae.

Further, of the above-described known compounds, those effective on both viruses of Herpesviridae and influenza viruses are limited to the glycyrrhetic acid derivatives and the compounds of Japanese Patent Application No. 306597/91 both discovered by the same inventors as the present invention.

The recent studies on antiviral agents have achieved a remarkable development. In particular, various nucleic acid type antiviral agents exemplified by acyclovir have been recognized to have excellent clinical efficacy on infectious diseases caused by herpes simplex virus (HSV) or herpes zoster virus. However, infectious diseases caused by resistant herpes viruses such as thymidine kinase-deficient virus have already posed a serious problem (see Oral Surg. Med. Oral Pathol., Vol. 67, pp. 427-432 (1989)). In addition, these nucleic acid type antiviral agents are generally ineffective on RNA viruses exemplified by influenza virus.

Known antiviral compounds effective on DNA viruses other than the nucleic acid type include phosphonoformate (PFA) (Nippon Rinsho, Vol. 47 (2), pp. 390-394 (1989)), phosphonoacetate (PAA) (ibid., Vol. 47 (2), pp. 390-394 (1989)), and cicloxolone (Journal of Antimicrobial Chemotherapy, Vol. 18, Suppl. B., pp. 185-200 (1986)). However, PFA and PAA involve the problem of side effects, such as disturbances of renal functions or development of anemia; and the antiviral activity of cicloxolone is not sufficiently high to be used for treatment.

Known antiviral compounds effective on RNA viruses other than the nucleic acid type derivatives include amantadine (Shonika Shinryo, Vol. 54(4), pp. 988-994 (1991)), rimantadine (ibid., Vol. 54(4), pp. 988-994 (1991)), and LY 253963 (a thiadiazole derivative) (ibid., Vol. 54(4), pp. 988-994 (1991)). Amantadine is, though effective on influenza A virus, ineffective on influenza B virus and also has a problem of side effects on the central nervous system (CNS). Rimantadine shows a considerable improvement over amantadine but still involves the problem of side effects on the CNS. Further, LY 253963 has received confirmation to its anti-influenza virus activity in animals but not to the clinical efficacy.

Known antiviral agents effective on retroviruses exemplified by AIDS virus (HIV) include azidodeoxythymidine (AZT) and dideoxyinosine (DDI) (Shonika Shinryo, Vol. 54(4), pp. 981-987 (1991)). These antiviral agents retard the outbreak of AIDS but are not expected to bring complete healing and, in addition, have been pointed out to involve medullary inhibition as a side effect.

Patients suffering from immunodeficiency caused by organ transplantation, chemotherapy for cancers, or HIV infection tend to increase in recent years, giving rise to a serious medical problem. A great variety of virus infectious diseases occur in such patients and very often cannot be treated with conventionally available antiviral drugs. Hence, great expectations have been held for development of more excellent antiviral agents.

## SUMMARY OF THE INVENTION

An object of the present invention is to provide an antiviral agent having excellent effects on DNA viruses, RNA viruses, and retroviruses.

Another object of the present invention is to provide an antiviral agent having a chemical structure different from the conventional antiviral substances in expectation of effectiveness on viruses resistant to the conventional antiviral substances.

The inventors previously elucidated that glycyrrhetic acid derivatives having various substituents at the 30-position thereof exhibit excellent antiviral activities (Japanese Patent Application No. 127271/91). In particular, a glycyrrhetic acid derivative with a phenylpiperazine derivative incorporated into the 30-position thereof, i.e., 1-[3β-(3-carboxypropanoyloxy)-18β-olean-12-en-30-oyl]-4-(o-methoxyphenyl)piperazine, has been recognized to have an excellent antiviral activity and high safety.

For the purpose of further elucidating the significance of phenylpiperazine group introduced to the 30-position of glycyrrhetic acid or an analogous group thereof, the inventors continued their study on the antiviral activities and cytotoxicity of known substituted or unsubstituted phenylpiperazine, phenylpiperidine, phenylpyrrolidine, phenyltetrahydroimidazole, phenylhomopiperazine, phenylaziridine, phenylazetidine, phenyldiazetidine, or phenylperhydroazepine. As a result, it was unexpectedly found that a number of these compounds exhibit a broad antiviral spectrum against various DNA viruses, RNA viruses, and retroviruses, such as herpes simplex virus (HSV-1, HSV-2), vaccinia virus, herpes zoster virus, cytomegalovirus, influenza virus, type B hepatitis virus (HBV), AIDS virus (HIV), etc. and are effective in either topical administration or systemic administration.

Now that the antiviral activities of the substituted or unsubstituted phenylpiperazine, phenylpiperidine, phenylpyrrolidine, phenyltetrahydroimidazole, and phenylhomopiperazine per se and, in addition, substituted or unsubstituted phenylaziridine, phenylazetidine, phenyldiazetidine, and phenylperhydroazepine per se had been revealed, the inventors examined antiviral activities of known compounds containing, in the structure thereof, a substituent derived from these compounds. As a result, it was found that some of them possess potent antiviral activities as disclosed in Japanese Patent Application No. 306597/91.

The inventors have further conducted extensive investigations with the purpose of obtaining further improved antiviral activity in mind based on the above-mentioned findings, designed novel compounds, and examined their antiviral activities. It has now been found as a result that some of the novel compounds exhibit strong antiviral activities and, at the same time, have reduced cytotoxicity. The present invention has been completed based on this finding.

According to the inventors' finding, novel antiviral agents with improved antiviral activities can be designed by adding various chemical modifications to phenylpiperazine, phenylpiperidine, phenylpyrrolidine, phenyltetrahydroimidazole, phenylhomopiperazine, phenylaziridine, phenylazetidine, phenyldiazetidine, or phenylperhydroazepine as a basic skeleton.

The inventors have also reached the conclusion that certain known pharmacologically active or inactive compounds or novel pharmacologically active or inactive compounds can be endowed with antiviral activities or their antiviral activities can be enhanced by introduction of substituted or unsubstituted phenylpiperazine, phenylpiperidine, phenylpyrrolidine, phenyltetrahydroimidazole, phenylhomopiperazine, phenylaziridine, phenylazetidine, phenyldiazetidine, or phenylperhydroazepine.

While not limiting, compounds into which substituted or unsubstituted phenylpiperazine, phenylpiperidine, phenylpyrrolidine, phenyltetrahydroimidazole, phenylhomopiperazine, phenylaziridine, phenylazetidine, phenyldiazetidine, or phenylperhydroazepine may be introduced chemically include nucleic acid type antiviral substances typically exemplified by acyclovir and AraA; non-steroid antiinflammatory substances used as therapeutic adjuncts in the treatment of viral infectious diseases; antibacterial or antimicrobial substances, such as sulfa preparations, β-lactam preparations, and pyridonecarboxylic acids; and naturally occurring substances or substances of living body origin, such as fatty acids, amino acids, peptides, sugars, steroids, and flavones.

The active ingredient of the antiviral agents according to the present invention, i.e., phenylpiperazine derivatives, phenylpiperidine derivatives, phenylpyrrolidine derivatives, phenyltetrahydroimidazole derivatives, phenylhomopiperazine derivatives, phenylaziridine derivatives, phenylazetidine derivatives, phenyldiazetidine derivatives, and phenylperhydroazepine derivatives, include compounds represented by formula (I) and pharmaceutically acceptable salts thereof.

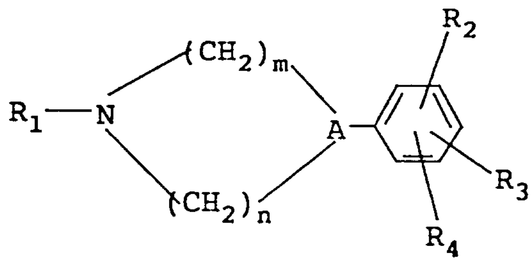

(I)

wherein $R_1$ represents a substituted or unsubstituted alkyl group, a substituted or unsubstituted acyl group, a substituted or unsubstituted arylsulfonyl group, a substituted or unsubstituted alkylsulfonyl group, a substituted or unsubstituted phenyl group, or a substituted or unsubstituted heterocyclic group, preferably fatty acid (e.g., octanoyl or linolenoil glycyrrhizic acid, eburnamonine, quinolone carboxylic acid, and derivatives thereof; $R_2$, $R_3$, and $R_4$, which may be the same or different, each represent a hydrogen atom, an amino group, a substituted or unsubstituted alkylamino group, an acylamino group, a substituted or unsubstituted alkyl group, a hydroxyl group, a substituted or unsubstituted alkyloxy group, a halogen atom, a carboxyl group, a substituted or unsubstituted alkylcarbonyl group, a substituted or unsubstituted alkoxycarbonyl group, a substituted or unsubstituted aryloxycarbonyl group, a substituted or unsubstituted carbamoyl group, a nitro group, a cyano group, a thiol group, a substituted or unsubstituted alkylthio group, a substituted or unsubstituted phenyl group, or a substituted or unsubstituted heterocyclic group, preferably a chlorine atom, a fluorine atom, a bromine atom, a trifluoromethyl group, a cyano group, a nitro group, a methoxy group, a hydroxyl group, an alkyl group having 1 to 5 carbon atoms or an acyl group having 1 to 5 carbon atoms; A represents a nitrogen group or a methylene group; m represents 0 or a natural number (preferably 1 to 4); n represents a natural number (preferably 1 to 4); and the carbon atoms of $R_1$ to $R_4$ are preferably 2 to 20.

## DETAILED DESCRIPTION OF THE INVENTION

The terminology "may be substituted" means that the group may be substituted with an amino group, a formyl group, a hydroxyl group, an alkoxy group, an aryloxy group, a halogen atom, a nitro group, a cyano group, a thiol group, an alkylthio group, an arylthio group, an acyl group, a carbamoyl group, an alkylsilyl group, an arylsilyl group, an alkyloxycarbonyl group, an aryloxycarbonyl group, an alkylsulfonyl group, an arylsulfonyl group, an alkylsulfinyl group, an arylsulfinyl group, or an acid radical, e.g., of phosphoric acid, phosphonic acid, phosphinic acid, phosphenic acid, sulfuric acid, sulfinic acid, sulfenic acid, or boric acid, or an ester thereof.

The terminology "heterocyclic group" means, for example, a pyridine ring, a piperidine ring, a pyrazine ring, a piperazine ring, a pyrrole ring, a pyrrolidine ring, an oxazole ring, an imidazole ring, a morpholine ring, a diazole ring, a triazole ring, a tetrazole ring, a thiazole ring, a thiadiazole ring, etc., each of which may be condensed with a benzene ring or with each other.

Specific but non-limiting examples of the compounds represented by formula (I) are 1-acetyl-4-phenylpiperazine, 1-acetyl-4-(o-, m- or p-chlorophenyl)piperazine, 1-acetyl-4-(o-, m- or p-trifluoromethylphenyl)-piperazine, 1-phenyl-4-decanoylpiperazine, 1-(o-, m- or p-chlorophenyl)-4-decanoylpiperazine, 1-(o-, m- or p-trifluoromethylphenyl)-4-decanoylpiperazine, 1-octanoyl-4-phenylpiperazine, 1-(o-, m- or p-chlorophenyl)-4-octanoylpiperazine, 1-(o-, m- or p-trifluoromethylphenyl)-4-octanoylpiperazine, 1-phenyl-4-octadecanoylpiperazine, 1-(o-, m- or p-chlorophenyl)-4-octadecanoylpiperazine, 1-(o-, m- or p-trifluoromethylphenyl)-4-octadecanoylpiperazine, 1-[(Z,Z)-9,12-octadecadienoyl]-4-phenylpiperazine, 1-(o-, m- or p-chlorophenyl)-4-[(Z,Z)-9,12-octadecadienoyl]piperazine, 1-(o-, m- or p-trifluoromethylphenyl)-4-[-(Z,Z)-9,12-octadecadienoyl]piperazine, 1-(5β-cholan-24-oyl)-4-phenylpiperazine, 1-(o-, m- or p-chlorophenyl)-4-(5β-cholan-24-oyl)piperazine, 1-(5β-cholan-24-oil)-4-(o-, m- or p-trifluoromethylphenyl)-piperazine, 1-(4-aminophenylsulfonyl)-4-phenylpiperazine, 1-(4-aminophenylsulfonyl)-4-(o-, m- or p-chlorophenyl)piperazine, 1-(4-aminophenylsulfonyl)-4-(o-, m- or p-trifluoromethylphenyl)piperazine, 1-ethyl-4-phenylpiperazine, 1-(o-, m- or p-chlorophenyl)-4-ethylpiperazine, 1-ethyl-4-(o-, m- or p-trifluoromethylphenyl)piperazine, 1-phenyl-4-(2-pyrrolidon-1-yl)acetylpiperazine, 1-(o-, m- or p-chlorophenyl)-4-(2-pyrrolidon-1-yl)acetylpiperazine,1-(o-, m- or p-trifluoromethylphenyl)-4-(2-pyrrolidon-1-yl)acetylpiperazine, 1-(2-acetoxybenzoyl)-4-phenylpiperazine, 1-(2-acetoxybenzoyl)-4-(o-,m-or p-chlorophenyl)piperazine, 1-(2-acetoxybenzoyl)-4-(o-, m- or p-trifluoromethylphenyl)piperazine, 1-(2-hydroxybenzoyl)-4-phenylpiperazine, 1-(2-hydroxybenzoyl)-4-(o-,m-or p-chlorophenyl)piperazine, 1-(2-hydroxybenzoyl)-4-(o-, m- or p-trifluoromethylphenyl)piperazine, 1-(1-azabicyclo[3.3.0]octan-5-yl)-4-phenylpiperazine, 1-(1-azabicyclo[3.3.0]-octan-5-yl)-4-(o-, m- or p-chlorophenyl)piperazine, 1-(1-azabicyclo[3.3.0]octan-5-yl)-4-(o-, m- or p-trifluoromethylphenyl)piperazine, L-glutamic acid α-(4-phenylpiperazine)amide, L-glutamic acid α-[4-(o-, m- or p-chlorophenyl)piperazine]amide, L-glutamic acid α-[4-(o-, m-orp-trifluoromethylphenyl)piperazine]amide, 1-(3β-hydroxy-18β-olean-12-en-30-oyl)-4-phenylpiperazine, 1-(3β-hydroxy-18β-olean-12-en-30-oyl)-4-(o-, m- or p-chlorophenyl)piperazine, 1-(o-, m- or p-trifluoromethylphenyl)-4-(3β-hydroxy-18β-olean-12-en-30-oyl)-piperazine, and pharmaceutically acceptable salts thereof, preferably 7-[4-(2-chlorophenyl)-1-piperazinyl]-1-ethyl-6-fluoro-1,4-dihydro-4-oxoquinoline-3-carboxylic acid, 1-(2-chlorophenyl)-4-[(Z,Z)-9,12-octadecadienoyl]piperazine, 1-(2-chlorophenyl)-4-octanoylpiperazine, 1-(4-aminosulfonylphenyl)-4-(2-chlorophenyl)piperazine, 1-(2-trifluoromethylphenyl)-4-octanoylpiperazine, 1-ethyl-6-fluoro-7-[4-(2-trifluoromethylphenyl)-1-piperazinyl]-1,4-dihydro-4-oxoquinoline-3-carboxylic acid, N-[4-(2-chlorophenyl)-piperazine-1-yl]ethyl-(3α,16α)-eburunamenine-14-caboxamide or (3α,16α)-eburunamenine-14-carboxylic acid 2-[4-(2-chlorophenyl)piperazine-1-yl]ethyl ester.

The pharmaceutically acceptable salt of the compound represented by formula (I) includes inorganic acid salts (e.g., salts formed with inorganic acid such as hydrochloric acid, hydrobromic acid and sulfuric acid) and organic acid salts (e.g., salts formed with organic acids such as acetic acid, fumaric acid, maleic acid, tartaric acid, succinic acid, malic acid and methanesulfonic acid), but is not limited thereto.

The compound represented by formula (I) and pharmaceutically acceptable salts thereof can be easily synthesized by the conventional methods. For example, acid halides react with phenylpiperazines, phenylpiperidines, phenylpyrrolidines, phenyltetrahydroimidazoles, or phenylhomopiperazines to give each desired compound through dehydrohalogenation. Carboxylic acids react with phenylpiperazines, or the compounds described above to give each desired compounds through dehydration using DCC (dicyclohexylcarbodiimide), EDC [1-(3-dimethylaminopropyl)-3-ethylcarbodiimide] and so on.

The compounds represented by formula (I) possess potent antiviral activities and are of low toxicity and may be provided in various dose forms.

Preparations for topical administration include solutions, ointments, creams, gels, rectal suppositories, vaginal suppositories, etc. for the skin or mucosa; and eye drops or eye ointments for eyes. These preparations can be obtained in a usual manner. A dose level of the active ingredient for humans depends on the kind of the active ingredient, the symptoms, the dose form, and the like. In general, the preparations for topical administration suitably contain the active ingredient in a concentration of from 0.01 to 10% by weight.

If desired, with the purpose of absorption acceleration, an absorption accelerator, such as gallic acid, saponin, polyoxyethylene higher alcohol ethers, polyethylene glycol, dimethyl sulfoxide (DMSO), laurocapram, etc. may be added to the preparations.

Preparations for oral administration include solutions, tablets, capsules, granules, fine granules, buccal tablets, troches, and so on. These preparations may be prepared in a usual manner. If desired, an absorption accelerator, such as gallic acid, saponin, polyoxyethylene higher alcohol ethers, etc. may be added to the oral preparations. A dose level of the active ingredient for humans depends on the kind of the active ingredient, the symptoms, the dose form, and the like. In general, a suitable daily dose is from 100 to 3000 mg. The antiviral agent according to this invention can be prepared into the dose form of injection. In such injection administration, a suitable daily dose is from 30 to 1000 mg.

The followings are the examples of the formulations and these are purely illustrative and in no way to be interpreted as restrictive.

FORMULATION EXAMPLE 1 (Ointments)

A typical ointment containing the following ingredients is prepared in a conventional manner and is filled into aluminum tubes.

| | |
|---|---|
| Compound of Example 5<br>White petrolatum | 3 g<br>proper amount |
| | 100 g |

FORMULATION EXAMPLE 2 (Oral ointments)

A typical oral ointment containing the following ingredients is prepared in a conventional manner and is filled into aluminum tubes.

| | |
|---|---|
| Compound of Example 8 | 0.3 g |
| Carboxymethylcellulose (Na) | 3.1 g |
| Liquid paraffin | 3.1 g |
| White petrolatum | 1.2 g |
| Plastibase (liquid paraffine: 95%, polyethylene resin: 5%) | proper amount |
| | 10 g |

FORMULATION EXAMPLE 3 (Capsules)

A typical capsule containing the following ingredients is prepared in a conventional manner.

| | |
|---|---|
| Compound of Example 9 | 100 mg |
| Magnesium stearate | 5 mg |
| Lactose | proper amount |
| | 150 mg |

FORMULATION EXAMPLE 4 (Tablets)

A typical capsule containing the following ingredients is prepared in a conventional manner.

| | |
|---|---|
| Compound of Example 11 | 100 mg |
| Sodium lauryl sulfate | 10 mg |
| Magnesium stearate | 5 mg |
| Polyvinyl pyrrolidone K30 | 11 mg |
| Carboxymethylcellulose (Ca) | 7 mg |
| Lactose | 60 mg |
| Corn starch | proper amount |
| | 210 mg |

The present invention is now illustrated in greater detail with reference to Examples and Test Examples, but it should be understood that the present invention is not to be construed as being limited thereto.

EXAMPLE 1

1-(2-Chlorophenyl)-4-(2-pyrrolidon-1-yl)acetylpiperazine

A mixture of 1-(2-chlorophenyl)piperazine (984 mg) and methyl 2-pyrrolidon-1-acetate (802 mg) was stirred at 90°C for 24 hours under argon atmosphere. The reaction mixture was purified by column chromatography on silica gel (Wakogel C-200) using ether/methanol (10:1) as eluent to give 1.24 g (77.1%) of the title compound as a colorless oil.
MS Spectrum m/z:
EI/DI; 323, 321 ($M^+$), 168, 166 (base).
$^1$H-NMR Spectrum ($CDCl_3$) $\delta$ ppm:
2.09 (2H, t, J=7.8Hz, pyrrolidone H), 2.45 (2H, t, J=7.8Hz, pyrrolidone H), 3.01-3.07 (4H, m, piperazine H), 3.54 (2H, t, J=7.8Hz, pyrrolidone H), 3.65 (4H, t, J=4.9Hz, piperazine H), 4.16 (2H, s, $N(C=O)CH_2N$), 6.99-7.05 (2H, m, ArH), 7.21-7.27 (1H, m, ArH), 7.36-7.40 (1H, m, ArH).
IR Spectrum (KBr) $\nu$ $cm^{-1}$:

1685 (C = O), 1659 (C = O of pyrrolidone).

EXAMPLE 2

1-(2-Acetoxybenzoyl)-4-(2-chlorophenyl)piperazine

To a stirred solution of 1-(2-chlorophenyl)piperazine (3.25 g) and triethylamine (1.67 g) in dichloromethane (60.0 ml) was added dropwise O-acetylsalicyloyl chloride (3.20 g) at 0 to 5 ° C under argon atmosphere. After additional stirring for 30 minutes, the reaction mixture was concentrated in vacuo. The residue was purified by column chromatography on silica gel (Wakogel C-200) using ether/n-hexane (2:1) as eluent to give 4.90 g (91.0%) of the title compound as a colorless oil.
Melting point: 115-116 ° C.
MS Spectrum m/z:
EI/DI; 360, 358 ($M^+$), 166 (base).
$^1$H-NMR Spectrum ($CDCl_3$) $\delta$ ppm:
2.30 (3H, s, $CH_3$), 2.95-2.98, 3.02-3.18, 3.48-3.52 and 3.90-4.03 (8H, each m, piperazine H), 6.99-7.03 (2H, m, ArH), 7.16-7.44 (6H, m, ArH).
IR Spectrum (KBr) $\nu$ $cm^{-1}$:
1771 (C = O of acetoxy), 1632 (C = O).

EXAMPLE 3

1-(2-Chlorophenyl)-4-(2-hydroxybenzoyl)piperazine

To a stirred solution of the title compound (2.52 g) of Example 2 in 1,4-dioxane (70.0 ml) was added a solution of 5%-sodium hydroxide in methanol (56.2 ml) at 10 to 20 ° C. After additional stirring for 10 minutes, dichloromethane (1 ℓ) and brine (500 ml) were added to the mixture. The organic layer was separated, washed with brine (500 ml), dried over potassium carbonate, concentrated in vacuo to give 2.20 g (98.9%) of the title compound as colorless prisms.
Melting point: 166-167 ° C.
MS Spectrum m/z:
EL/DI; 318, 316 ($M^+$), 179 (base).
$^1$H-NMR Spectrum ($CDCl_3$) $\delta$ ppm:
3.08-3.12 and 3.92-3.96 (8H, each m, piperazine H), 6.85-6.91 (1H, m, ArH), 7.00-7.05 (3H, m, ArH), 7.21-7.41 (4H, m, ArH), 9.63 (1H, s, OH).
IR Spectrum (KBr) $\nu$ $cm^{-1}$:
1610 (C = O).

EXAMPLE 4

1-(1-Azabicyclo[3.3.0]octan-5-yl)acetyl-4-(2-chlorophenyl)piperazine

A mixture of 5-methoxycarbonylmethyl-1-azabicyclo[3.3.0]octane (1.38 g) and 1-(2-chlorophenyl)-piperazine (1.78 g) was stirred at 120 ° C for 60 hours under argon atmosphere. The reaction mixture was purified by column chromatography on silica gel (Wakogel C-200) using dichloromethane/methanol (5:1) as eluent to give 1.31 g (50.0%) of the title compound as a colorless oil.
MS Spectrum m/z:
EL/DI; 349, 347 ($M^+$), 110 (base).
$^1$H-NMR Spectrum ($CDCl_3$) $\delta$ ppm:
1.65-2.05 (8H, m, $CH_2$), 2.45-2.61 (2H, m, $CH_2$), 2.53 (2H, s, $CH_2$(C = O)), 2.94-3.04 (6H, m, piperazine H and $CH_2$), 3.69-3.81 (4H, m, piperazine H), 6.97-7.02 (2H, m, ArH), 7.20-7.27 (1H, m, ArH), 7.36-7.40 (1H, m, ArH).
IR Spectrum (KBr) $\nu$ $cm^{-1}$:
1635 (C = O).

EXAMPLE 5

7-[4-(2-Chlorophenyl)-1-piperazinyl]-1-ethyl-6-fluoro-1,4-dihydro-4-oxoquinoline-3-carboxylic acid

A mixture of 1-ethyl-6,7-difluoro-1,4-dihydro-4-oxoquinoline-3-carboxylic acid (127 mg), 1-(2-chlorophenyl)piperazine (295 mg) and acetonitrile (10.0 ml) was refluxed for 24 hours under argon atmosphere. After cooling, the precipitate was filtered to give 160 mg (74.4%) of the title compound as colorless prisms.
Melting point: 246-247 °C
MS Spectrum m/z:
EL/DI; 431, 429 ($M^+$), 168, 166 (base).
$^1$H-NMR Spectrum ($CDCl_3$) $\delta$ ppm:
1.44 (3H, t, J = 7.3Hz, $CH_2\underline{CH_3}$), 3.16-3.25 and 3.43-3.56 (8H, each m, piperazine H), 4.62 (2H, q, J = 7.3Hz, $\underline{CH_2}CH_3$), 7.06-7.12, 7.24-7.38, 7.44-7.47 (5H, each m, other ArH), 7.95 (1H, d, J = 13.2Hz, $C_5$-H), 8.97 (1H, s, $C_2$-H).
IR Spectrum (KBr) $\nu$ $cm^{-1}$:
1723 (C = O of COOH), 1627 (C = O).

EXAMPLE 6

1-(2-Chlorophenyl)-4-methylsulfonylpiperazine

Using a similar procedure to that described in Example 2 except that methanesulfonyl chloride was used instead of O-acetylsalicyloyl chloride, the title compound [2.35 g (84.1%)], which was crystallized from ether, was obtained as colorless prisms starting from 1-(2-chlorophenyl)piperazine (1.28 g).
Melting point: 153-154 °C.
MS Spectrum m/z:
EL/DI; 276, 274 ($M^+$), 197, 195 (base).
$^1$H-NMR Spectrum ($CDCl_3$) $\delta$ ppm:
2.85 (3H, s, $CH_3$), 3.14-3.17, 3.40-3.44 (8H, each m, piperazine H), 7.01-7.06 (2H, m, ArH), 7.23-7.29 (1H, m, ArH), 7.36-7.40 (1H, m, ArH).
IR Spectrum (KBr) $\nu$ $cm^{-1}$:
1346, 1324, 1168, 1153 ($SO_2$).

EXAMPLE 7

1-(2-Chlorophenyl)-4-octadecanoylpiperazine

Using a similar procedure to that described in Example 2 except that stearoyl chloride was used instead of O-acetylsalicyloyl chloride and ether/hexane (1:4) was used as eluent on a column chromatography, the title compound [5.65 g (92.4%)] was obtained as colorless crystals starting from 1-(2-chlorophenyl)-piperazine (2.65 g).
Melting point: 54-55 °C.
MS Spectrum m/z:
EI/DI; 464, 462 ($M^+$), 156, 154 (base).
$^1$H-NMR Spectrum ($CDCl_3$) $\delta$ ppm:
0.88 (3H, t, J = 6.8Hz, $CH_3$), 1.15-1.40 and 1.56-1.72 (30H, m, $CH_2$), 2.37 (2H, dd, J = 7.3 and 7.8Hz, $CH_2$-(C = O)), 2.94-3.06 (4H, m, piperazine H), 3.58-3.84 (4H, m, piperazine H), 6.97-7.04 (2H, m, ArH), 7.19-7.28 (1H, m, ArH), 7.35-7.42 (1H, m, ArH).
IR Spectrum (KBr) $\nu$ $cm^{-1}$:
2924, 2852 ($CH_2$), 1650 (C = O), 1589, 1481 (Ar).

EXAMPLE 8

1-(2-Chlorophenyl)-4-[(Z,Z)-9,12-octadecadienoyl]piperazine

Using a similar procedure to that described in Example 2 except that linoleoyl chloride was used instead of O-acetylsalicyloyl chloride and ether/hexane (1:4) was used as eluent on a column chromatography, the title compound [1.54 (quantitative yield)] was obtained as a pale yellow oil starting from 1-(2-chlorophenyl)piperazine (691 mg).
MS Spectrum m/z:
EI/DI; 460, 458 ($M^+$), 168, 166 (base).
$^1$H-NMR Spectrum ($CDCl_3$) $\delta$ ppm:
0.89 (3H, t, J = 7.3Hz, $CH_3$), 1.22-1.44 and 1.58-1.73 (16H, m, $CH_2$), 1.95-2.13 (4H, m, $C_8$-H, $C_{14}$-H), 2.37 (2H, t, J = 7.8Hz, $CH_2$(C = O)), 2.77 (2H, t, J = 5.8Hz, $C_{11}$-H), 2.88-3.08 (4H, m, piperazine H), 3.58-3.86 (4H, m, piperazine H), 5.27-5.44 (4H, m, olefine H), 6.96-7.06 (2H, m, ArH), 7.19-7.30 (1H, m, ArH), 7.36-7.42 (1H, m, ArH).
IR Spectrum (neat) $\nu$ $cm^{-1}$:
3008 (ArH), 2926, 2854 ($CH_2$), 1651 (C = O), 1589, 1480 (Ar), 1456, 1441 (C = C).

EXAMPLE 9

1-(2-Chlorophenyl)-4-octanoylpiperazine

Using a similar procedure to that described in Example 2 except that octanoyl chloride was used instead of O-acetylsalicyloyl chloride and ether/hexane (1:4) was used as eluent on a column chromatography, the title compound [2.59 g (80.0%)] was obtained as a pale yellow oil starting from 1-(2-chlorophenyl)piperazine (2.00 g).
MS Spectrum m/z:
EI/DI; 324, 322 ($M^+$), 168, 166 (base).
$^1$H-NMR Spectrum ($CDCl_3$) $\delta$ ppm:
0.88 (3H, t, J = 6.8Hz, $CH_3$), 1.2-1.45 and 1.6-1.72 (10H, m, $CH_2$), 2.37 (2H, dd, J = 8.3, 7.3Hz, $CH_2$C( = O)), 2.95-3.07 (4H, m, piperazine H), 3.60-3.85 (4H, m, piperazine H), 6.97-7.08 (2H, m, ArH), 7.20-7.28 (1H, m, ArH), 7.36-7.42 (1H, m, ArH).
IR Spectrum (neat) $\nu$ $cm^{-1}$:
2926, 2855 ($CH_2$), 1649 (C = O), 1589, 1480 (Ar).

EXAMPLE 10

1-(2-Chlorophenyl)-4-(5$\beta$-cholan-24-oyl)piperazine

To a stirred solution of 5$\beta$-cholanic acid (180.3 mg) and triethylamine (0.070 ml) in dichloromethane (5.00 ml) under argon atmosphere was added dropwise ethyl chlorocarbonate (0.048 ml) at 0 to 5 °C, and the mixture was stirred at the same temperature for 20 minutes. To this mixture was added a solution of 1-(2-chlorophenyl)piperazine (108.0 mg) in dichloromethane (2.00 ml) at 0 to 5 °C and the mixture was stirred for 1 hour. After evaporation, the residue was purified by column chromatography on silica gel (Wakogel C-200) using ether/n-hexane (1:5) as eluent to give 268.0 mg (99.4%) of the title compound as colorless prisms.
Melting point: 145-149 °C.
MS Spectrum m/z:
EI/DI; 540, 538 ($M^+$), 155, 153 (base).
$^1$H-NMR Spectrum ($CDCl_3$) $\delta$ ppm:
0.65 (3H, s, $CH_3$), 0.8-2.0 (34H, m, CH and $CH_2$), 0.91 (3H, s, $CH_3$), 0.96 (3H, d, J = 6.4Hz, $CH_3$), 2.20-2.47 (2H, m, $CH_2$(C = O)), 2.85-3.10 (4H, m, piperazine H), 3.55-3.83 (4H, m, piperazine H), 6.97-7.06 (2H, m, ArH), 7.20-7.28 (1H, m, ArH), 7.36-7.42 (1H, m, ArH).
IR Spectrum (KBr) $\nu$ $cm^{-1}$:
2928, 2860 ($CH_2$), 1648 (C = O), 1589, 1480 (Ar).

EXAMPLE 11

1-(4-Aminosulfonylphenyl)-4-(2-chlorophenyl)piperazine

To a stirred solution of 1-(2-chlorophenyl)piperazine (1.97 g) and triethylamine (1.53 ml) in dichloromethane (25.0 ml) was added portionwise N-acetylsulfanilyl chloride (2.34 g) at -5 to 0 °C under argon atmosphere. After additional stirring for 30 minutes, the mixture was washed with dil.-hydrochloric acid, dried over sodium sulfate and concentrated in vacuo to give 3.90 g (98.9%) of 1-(4-acetylaminosulfonyl)-4-(2-chlorophenyl)piperazine. This intermediate (2.71 g) was dissolved in methanol (100 ml) under argon atmosphere, and 2N-sodium hydroxide (34.4 ml) was added. After refluxing for 84 hours, the reaction mixture was concentrated in vacuo. The residue was treated with water-dichloromethane (5/50 ml), and the organic layer was separated, dried over sodium sulfate and concentrated in vacuo. The residue was recrystallized from dichloromethane/n-hexane (5/5 ml) to give 2.24 g (92.5%) of the title compound as colorless plates.
Melting point: 150-152 °C.
MS Spectrum m/z:
EI/DI; 353, 351 ($M^+$), 197, 195 (base).
$^1$H-NMR Spectrum ($CDCl_3$) $\delta$ ppm:
3.10 (8H, brs, piperazine H), 4.16 (2H, brs, $NH_2$), 6.88 and 7.43 (each 2H, AABB, N-Ph-S), 6.75-7.35 (4H, m, ArH).
IR Spectrum (KBr) $\nu$ $cm^{-1}$:
3485, 3388 ($NH_2$), 1596, 1481 (Ar), 1318, 1157 ($SO_2$).

EXAMPLE 12

1-(2-Chlorophenyl)-4-ethylpiperazine

A mixture of 1-(2-chlorophenyl)piperazine (1.97 g), ethyl bromide (1.09 g), potassium carbonate (1.38 g) and acetone (10.0 ml) was stirred at 10 to 20 °C for 36 hours under argon atmosphere. The reaction mixture was evaporated and the residue was purified by vacuum-distillation to give 2.06 g (91.5%) of the title compound as a colorless oil.
Boiling point: 150-155 °C (2 mmHg).
MS Spectrum m/z:
EI/DI; 226, 224 ($M^+$, base).
$^1$H-NMR Spectrum ($CDCl_3$) $\delta$ ppm:
1.11 (3H, t, J = 7Hz, $CH_2\underline{CH_3}$), 2.46 (2H, q, J = 7Hz, $\underline{CH_2}CH_3$), 2.35-3.25 (8H, m, piperazine H), 6.5-7.35 (4H, m, ArH).
IR Spectrum (neat) $\nu$ $cm^{-1}$:
2970, 2879 ($CH_3$), 2946, 2815 ($CH_2$), 1589, 1481, 750 (Ar).

EXAMPLE 13

1-Acetyl-4-(2-chlorophenyl)piperazine

Using a similar procedure to that described in Example 2 except that acetyl chloride was used instead of O-acetylsalicyloyl chloride and ether/hexane (1:1) was used as eluent on a column chromatography, the title compound [2.58 g (95.6%)] was obtained as a colorless oil starting from 1-(2-chlorophenyl)piperazine (2.00 g).
MS Spectrum m/z:
EI/DI; 240, 238 ($M^+$), 168, 166 (base).
$^1$H-NMR Spectrum ($CDCl_3$) $\delta$ ppm:
2.11 (3H, s, $CH_3$), 2.85-3.15 (2H, m, piperazine H), 3.45-3.90 (2H, m, piperazine H), 6.7-7.4 (4H, m, ArH).
IR Spectrum (neat) $\nu$ $cm^{-1}$:
2910, 2858 ($CH_2$), 1650 (C = O), 1589, 1481 (Ar).

EXAMPLE 14

L-Glutamic acid $\alpha$-[4-(2-chlorophenyl)-1-piperazine]amide hydrochloride

A mixture of N-(t-butoxycarbonyl)-L-glutamic acid $\gamma$-benzyl ester (1.69 g), dicyclohexylcarbodiimide (1.03 g), 1-(2-(chlorophenyl)piperazine (985 mg) and dichloromethane (10.0 ml) was stirred at 0 to 20 °C for 2 hours under argon atmosphere. After filtration, the filtrate was concentrated in vacuo. The residue was dissolved in ethanol (20.0 ml) and hydrogenated with 10% palladium-carbon as a catalyst for 6 hours. After the reaction mixture was filtered, the filtrate was evaporated, and the residue was purified by column chromatography on silica gel (Wakogel C-200) using ether/n-hexane (2:1) as eluent to give 700 mg (32.8%) of N-(t-butoxycarbonyl)-L-glutamic acid. This resulting intermediate (400 mg) was dissolved in 1,4-dioxane

(23.4 ml) containing hydrogen chloride (0.8 N) and stirred for 24 hours under argon atmosphere. The precipitate was filtered to give 338 mg (99.3%) of the title compound as colorless prisms.
Melting point: 132-136 °C.
MS Spectrum m/z:
FAB; 328, 326 [$(M+H)^+$], 185 (base).
$^1$H-NMR Spectrum ($CD_3OD$) δ ppm:
2.00-2.30 (2H, m, $\underline{CH_2}CH_2COOH$), 2.50-2.66 (2H, m, $CH_2\underline{CH_2}COOH$), 3.15-3.37 (4H, m, piperazine H), 3.56-4.00 (4H, m, piperazine H), 4.56-4.66 (1H, m, CH), 7.21-7.23 (1H, m, ArH), 7.29-7.41 (2H, m, ArH), 7.44-7.51 (1H, m, ArH).
IR Spectrum (KBr) ν $cm^{-1}$:
3000-2500 (COOH and $NH_3^+$), 1717 (C=O of COOH), 1647 (C=O of amide).

EXAMPLE 15

1-(3β-Hydroxy-18β-olean-12-en-30-oyl)-4-(2-chlorophenyl)piperazine

To a stirred solution of 1-(3β-hydroxy-18β-olean-12-en-30-oyl)chloride in dichloromethane (20 ml) was added dropwise a solution of 1-(2-chlorophenyl)piperazine (799 mg) in dichloromethane (6.00 ml). After stirring at 20 to 25 °C for 1 hour, the reaction mixture was washed with water, dried over sodium sulfate, concentrated in vacuo. The residue was dissolved in 1,4-dioxane and a solution of 10%-potassium hydroxide in methanol (12.0 ml) was added. After stirring at 20 to 25 °C for 15 hours, the reaction mixture was poured into water (40.0 ml) and the resulting precipitate was filtered to give 2.04 g (90.8%) of the title compound as a colorless crystals.
Melting point: 224-228 °C
MS Spectrum m/z:
EI/DI; 636, 634 ($M^+$), 189 (base).
$^1$H-NMR Spectrum ($CDCl_3$) δ ppm:
0.8-2.1 (23H, m, $CH_2$ and CH), 0.79, 0.81, 0.94, 0.96, 1.00, 1.14, 1.23 (3H×7, each s, $CH_3$), 3.03 (4H, m, piperazine H), 3.21 (1H, m, $C_3$-H), 3.83 (4H, m, piperazine H), 5.33 (1H, t, J=3.4Hz, $C_{12}$-H), 7.02 (2H, m, ArH), 7.21 (1H, m, ArH), 7.37 (1H, m, ArH).
IR Spectrum (KBr) ν $cm^{-1}$:
2946 ($CH_2$), 1622 (C=O), 1590, 1480 (Ar).

EXAMPLE 16

1-(2-Trifluoromethylphenyl)-4-octanoylpiperazine

Using a similar method to that described in Example 9 except that 1-(2-trifluoromethylphenyl)piperazine (1.15 g) was used instead of 1-(2-chlorophenyl)piperazine and ether/n-hexane (1:2) was used as eluant on a column chromatography, the title compound [1.71 g (97.5%)] was obtained as a yellowish crystals.
Melting point: 41-42 °C.
MS Spectrum m/z:
EI/DI; 356 ($M^+$), 200 (base).
$^1$H-NMR Spectrum ($CDCl_3$) δ ppm:
0.89 (3H, t, J=6.8Hz, $CH_3$), 1.2-1.4 (8H, m, $CH_2$), 1.6-1.75 (2H, m, $CH_2CH_2$-C(=O)N), 2.3-2.4 (2H, m, $CH_2$-C(=O)N), 2.8-2.95 (4H, m, piperazine H), 3.55-3.65 (2H, m, piperazine H), 3.7-3.8 (2H, m, piperazine H), 7.22-7.34 (2H, m, ArH), 7.20-7.53 (1H, t, J=7.8Hz, ArH), 7.65 (1H, t, J=7.8Hz, ArH).
IR Spectrum (KBr) ν $cm^{-1}$:
1646 (C=O).

EXAMPLE 17

1-(2-Trifluoromethylphenyl)-4-[(Z,Z)-9,12-octadecadienoyl]piperazine

Using a similar method to that described in Example 8 except that 1-(2-trifluoromethylphenyl)piperazine (2.65 g) was used instead of 1-(2-chlorophenyl)piperazine, the title compound [5.65 g (92.4%)] was obtained as colorless crystals.
MS Spectrum m/z:

EI/DI; 492 ($M^+$, base).
$^1$H-NMR Spectrum ($CDCl_3$) $\delta$ ppm:
0.89 (3H, t, J = 6.8Hz, $CH_3$), 1.2-1.45 and 1.52-1.55 (16H, m, other $CH_2$), 2.0-2.15 (4H, m, $C_8$-H, $C_{14}$-H), 2.36 (2H, t, J = 7.3Hz, $CH_2$(C = O)), 2.77 (2H, t, J = 5.8Hz, $C_{11}$-H), 2.8-3.0 (4H, m, piperazine H), 3.55-3.7 (2H, m, piperazine H), 3.7-3.85 (2H, m, piperazine H), 5.27-5.45 (4H, m, olefine H), 7.2-7.4 (2H, m, ArH), 7.53 (1H, t, J = 7.8Hz, ArH), 7.65 (1H, dd, J = 7.8 and 1.5Hz, ArH).
IR Spectrum (KBr) $\nu$ $cm^{-1}$:
1652 (C = O).

EXAMPLE 18

4-(2-Trifluoromethylphenyl)-1-(3$\beta$-hydroxy-18$\beta$-olean-12-en-30-oyl)piperazine

Using a similar method to that described in Example 15 except that 1-(2-trifluoromethylphenyl)-piperazine (1.02 g) was used instead of 1-(2-chlorophenyl)piperazine, the title compound [1.84 g (99.8%)] was obtained as colorless crystals.
Melting point: 233-235 °C.
MS Spectrum m/z:
EI/DI; 668 ($M^+$, base).
$^1$H-NMR Spectrum ($CDCl_3$) $\delta$ ppm:
0.7-2.1 (23H, m, $CH_2$ and CH), 0.79, 0.82, 0.95, 0.97, 1.00, 1.14, 1.23 (3H×7, each s, $CH_3$), 2.85-3.0 (4H, m, piperazine H), 3.15-3.30 (1H, m, $C_3$-H), 3.7-3.85 (4H, m, piperazine H), 5.32-5.41 (1H, m, olefine H), 7.2-7.4 (2H, m, ArH), 7.53 (1H, t, J = 7.8Hz, ArH), 7.64 (1H, d, J = 7.8Hz, ArH).
IR Spectrum (KBr) $\nu$ $cm^{-1}$:
1636 (C = O), 1604, 1496 (C = C).

EXAMPLE 19

2-[4-(2-Trifluoromethylphenyl)-1-piperazinyl]-5,6,7-trimethoxy-1(2H)-phthalazinone

A mixture of 1-(2-trifluoromethylphenyl)piperazine (600 mg), 2-(4-bromobutyl)-5,6,7-trimethoxy-1-(2H)-phthalazinone (484 mg) and xylene (10.0 ml) was stirred at 100 °C for 18 hours under argon atmosphere. After filtration, the filtrate was evaporated under reduced pressure. The residue was purified by column chromatography (Wakogel C-200) using dichloromethane/methanol (10:1) as eluent to give 536 mg (79.0%) of the title compound as colorless prisms.
Melting point: 92-94 °C
MS Spectrum m/z:
EI/DI; 520 ($M^+$), 320 (base).
$^1$H-NMR Spectrum ($CDCl_3$) $\delta$ ppm:
1.53-1.70 and 1.80-1.88 (each 2H, each m, N-$CH_2CH_2CH_2CH_2$-N), 2.46 (2H, t, J = 7.3Hz, N-$CH_2CH_2CH_2CH_2$-N), 2.59 (4H, br, piperazine H), 2.94 (4H, t, J = 4.9Hz, piperazine H), 3.98, 4.02, 4.06 (3H×3, s×3, OMe×3), 4.27 (2H, t, J = 7.3Hz, N-$CH_2CH_2CH_2CH_2$-N),7.20 (1H, t, J = 7.8Hz, ArH), 7.36 (1H, d, J = 7.8Hz, ArH), 7.50 (1H, t, J = 7.8Hz, ArH), 7.61 (1H, d, J = 7.8Hz, ArH), 7.60 (1H, s, $C_8$-H), 8.33 (1H, s, $C_4$-H).
IR Spectrum (KBr) $\nu$ $cm^{-1}$:
1652 (C = O).

EXAMPLE 20

2-[4-(2-Trifluoromethylphenyl)-1-piperazinyl]hypoxanthine

A mixture of 2-bromohypoxanthine (1.02 g), 1-(2-trifluoromethylphenyl)piperazine (2.72 g), acetonitrile (30.0 ml) and water (15.0 ml) was refluxed for 10 hours. After cooling, the precipitate was filtered and dissolved in 2N-sodium hydroxide. After washing with ether (10 ml×5), the alkaline water layer was neutralized with acetic acid. The resulting precipitate was filtered and recrystallized from ethanol-water to give 1.31 g (75.8%) of the title compound as colorless prisms.
Melting point: >300 °C
MS Spectrum m/z:

EI/DI; 364 ($M^+$), 164 (base).
$^1$H-NMR Spectrum ($D_2O$/NaOD) δ ppm:
2.75-2.95 (4H, m, piperazine H), 3.52-3.72 (4H, m, piperazine H), 7.67 (1H, s, $C_8$-H), 7.15 (1H, t, J = 7.8Hz, ArH), 7.32 (1H, d, J = 7.8Hz, ArH), 7.49 (1H, t, J = 7.8Hz, ArH), 7.55 (1H, d, J = 7.8Hz, ArH).
IR Spectrum (KBr) ν $cm^{-1}$:
1685 (C = O).

EXAMPLE 21

2-[4-(2-Trifluoromethylphenyl)-1-piperazinyl]-7-(2-deoxy-α-and β-D-ribofuranosyl)hypoxanthine and 2-[4-(2-Trifluromethylphenyl)-1-piperazinyl]-9-(2-deoxy-α- and β-D-ribofuranosyl)hypoxanthine

A mixture of the compound of Example 20 (1.00 g), N,O-bistrimethylsilylacetoamide (BSA) (4.80 ml) and 1,2-dichloromethane (11.5 ml) was stirred for 2 hours. To this reaction mixture was added a solution of 1-chloro-3,5-di-p-toluoyl-2-deoxyribofuranose (1.05 g) in 1,2-dichloroethane (9.10 ml), then a solution of trimethylsilyltrifluoromethanesulfonate (TMSTF) (0.71 g) in benzene (2.30 ml), and stirred for 2 hours. After refluxed for 1 minute, the reaction mixture was treated with saturated sodium bicarbonate (50.0 ml) and dichloromethane (100 ml). The organic layer was separated, dried over sodium sulfate, and concentrated in vacuo. The residue was dissolved in methanol (50.0 ml), and silica gel (Wakogel C-200, 12 g) was added, and concentrated in vacuo. The silica gel, that absorbed the reaction mixture, was laid on non-treated silica gel (200 g) in column. The reaction mixture was purified by column chromatography using dichloromethane/methanol (20:1 to 8:1) as eluent to give 3 fractions. The first fraction was 7β-isomer [170 mg (12.9%)], the second was 7α-isomer [106 mg (8.03%)] and the third was the mixture of 9α-isomer and 9β-isomer [282 mg (21.4%)].

7β-isomer

Melting point: 183 ° C.
MS Spectrum m/z:
FAB; 481 [$(M + 1)^+$], 365 (base).
$^1$H-NMR Spectrum (DMSO-$d_6$) δ ppm:
2.23-2.36 and 2.41-2.54 (2H, m, $C_{2'}$-H), 2.86-3.0 (4H, m, piperazine H), 3.48-3.73 (6H, m, $C_{5'}$-H and piperazine H), 3.82-3.90 (1H, m, $C_{4'}$-H), 4.29-4.38 (1H, m, $C_{3'}$-N), 4.91 (1H, t, J = 5.4Hz, OH), 5.24 (1H, d, J = 4.4Hz, OH), 6.49 (1H, t, J = 8.3Hz, $C_{1'}$-H),7.37 (1H, t, J = 7.8Hz, ArH), 7.55-7.75 (3H, m, ArH), 8.33 (1H, s, $C_8$-H).
IR Spectrum (KBr) ν $cm^{-1}$:
3424 (OH), 1686 (C = O)

7α-isomer

Melting point: 212 ° C.
MS Spectrum m/z:
FAB: 481 [$(M + 1)^+$], 365 (base).
$^1$H-NMR Spectrum (DMSO-$d_6$) δ ppm:
2.18-2.28 and 2.6-2.75 (2H, m, $C_{2'}$-H), 2.85-3.0 (4H, m, piperazine H), 3.4-3.5 (1H, m, $C_{5'}$-H), 3.6-3.75 (4H, m, piperazine H), 4.12-4.20 (1H, m, $C_{4'}$-H), 4.25-4.35 (1H, m, $C_{3'}$-H), 4.75-4.85 (1H, m, OH), 5.25-5.35 (1H, m, OH), 6.53 (1H, dd, J = 2.4, 7.3Hz, $C_{1'}$-H), 7.36 (1H, t, J = 7.8Hz, ArH), 7.55-7.75 (3H, m, ArH), 8.27 (1H, s, $C_8$-H).
IR Spectrum (KBr) ν $cm^{-1}$:
3420 (OH), 1684 (C = O).

9β-isomer + 9α-isomer

Melting point: 160 ° C.
MS Spectrum m/z:
FAB: 481 [$(M + 1)^+$], 365 (base).
$^1$H-NMR Spectrum (DMSO-$d_6$) δ ppm:
2.15-2.32 (2H, m, $C_{2'}$-H), 2.94 (4H, m, piperazine H), 3.0-3.6 (1H, m, $C_{5'}$-H), 3.73 (4H, m, piperazine H),

3.75-3.85 and 4.03-4.12 (1H, m, $C_{4'}$-H), 4.21-4.31 and 4.31-4.41 (1H, m, $C_{3'}$-H), 4.6-5.6 (2H, m, OH), 6.12-6.22 (1H, m, $C_{1'}$-H), 7.31-7.40 (1H, m, ArH), 7.52-7.76 (3H, m, ArH), 7.97 and 8.07 (1H, each s, $C_8$-H).
IR Spectrum (KBr) $\nu$ $cm^{-1}$:
3398 (OH), 1688 (C=O).

EXAMPLE 22

1-(5-Methoxyindolyl-2-carbonyl)-4-(2-trifluoromethyl)piperazine

To a stirred suspension of 5-methoxyindolyl-2-carboxylic acid (956 mg), 1-(2-trifluoromethylphenyl)-piperazine (1.15 g) in dichloromethane (50.0 ml) was added dropwise a solution of EDC (1.05 g) in dichloromethane (40.0 ml) at 3 to 5°C. After additional stirring at 15 to 20°C for 2 hours, the reaction mixture was washed with 1N-hydrochloric acid (50.0 ml), dried over potassium carbonate, and concentrated in vacuo. The residue was recrystallized from ether/dichloromethane to give 1.87 g (92.7%) of the title compound as colorless prisms.
Melting point: 177-178°C.
MS Spectrum m/z:
EI/DI; 403 ($M^+$), 200 (base).
$^1$H-NMR Spectrum ($CDCl_3$) $\delta$ ppm:
3.00-3.05 (4H, m, piperazine H), 3.85 (3H, s, OMe), 4.05-4.15 (4H, m, piperazine H), 6.74 (1H, d, J=2.4Hz, ArH), 6.96 (1H, dd, J=8.8, 2.4Hz, ArH), 7.06 (1H, t, J=7.8Hz, ArH), 7.26-7.36 (3H, m, ArH), 7.55 (1H, t, J=7.8Hz, ArH), 7.67 (1H, d, J=7.8Hz, ArH), 9.45 (1H, br, NH).
IR Spectrum (KBr) $\nu$ $cm^{-1}$:
3260 (NH), 1602 (C=O).

EXAMPLE 23

1-Ethyl-6-fluoro-7-[4-(2-trifluoromethylphenyl)-1-piperazinyl]-1,4-dihydro-4-oxoquinoline-3-carboxylic acid

Using a similar method to that described in Example 5 except that 1-(2-chlorophenyl)piperazine was used instead of 1-(2-trifluoromethylphenyl)piperazine (691 mg), the title compound [428 mg (92.3%)] was obtained as colorless crystals.
Melting point: >300°C.
MS Spectrum m/z:
EI/DI; 463 ($M^+$), 419 (base).
$^1$H-NMR Spectrum (DMSO-$d_6$) $\delta$ ppm:
1.43 (3H, t, J=7.3Hz, $CH_2CH_3$), 3.05-3.15 (4H, m, piperazine H), 3.40-3.50 (4H, m, piperazine H), 4.64 (2H, q, J=7.3Hz, $CH_2CH_3$), 7.26-7.80 (5H, m, ArH), 7.96 (1H, d, J=13.7Hz, $C_5$-H), 9.00 (1H, s, $C_2$-H).
IR Spectrum (KBr) $\nu$ $cm^{-1}$:
1720 (COOH), 1628 (C=O).

EXAMPLE 24

2-[4-(4-(2-Trifluoromethylphenyl)-1-piperazinylacetoxy)phenyl]-5-hydroxy-3,7-dimethyl-4H-1-benzopyran-4-one

To a stirred solution of 1-(2-trifluoromethylphenyl)piperazine (1.15 g) and triethylamine (767 µl) in dichloromethane (25.0 ml) was added dropwise chloroacetyl chloride (438 µl) at 0 to 5°C. After additional stirring for 20 minutes, the reaction mixture was successively washed with water, 0.1N-hydrochloric acid and water, dried over potassium carbonate, and concentrated in vacuo to give 1.50 g (97.8%) of 1-chloroacetyl-4-(2-trifluoromethylphenyl)piperazine. A mixture of this resulting intermediate (230 mg), 5-hydroxy-2-(4-hydroxyphenyl)-3,7-dimethyl-4H-1-benzopyran-4-one (236 mg), potassium carbonate (2.07 g) and dimethyl formamide (DMF) was stirred at 60°C for 2 hours. The reaction mixture was poured into ice-water to give the crude title compound as precipitates. After column chromatography (Wakogel C-200) using dichloromethane/methanol (100:1) as eluent, this was finally purified by recrystallization from dichloromethane/methanol to give 280 mg (64.0%) of the pure title compound as yellowish crystals.
Melting point: 165-166°C.
MS Spectrum m/z:

EI/DI; 584 ($M^+$), 188 (base).
$^1$H-NMR Spectrum ($CDCl_3$) δ ppm:
2.91-2.96 (4H, m, piperazine H), 3.71-3.86 (4H, m, piperazine H), 3.87 (3H, s, OMe), 3.88 (3H, s, OMe), 4.84 (2H, s, $OCH_2CO$-), 6.36 (1H, d, J = 2.4Hz, $C_6$-H), 6.45 (1H, d, J = 2.4Hz, $C_8$-H), 7.10 (2H, dd, J = 6.8, 2.4Hz, ArH), 7.28-7.67 (4H, m, ArH), 8.10 (2H, dd, J = 6.8, 2.4Hz, ArH), 12.64 (1H, s, OH).
IR Spectrum (KBr) ν $cm^{-1}$:
3446 (OH), 1663 (C = O of amide), 1601 (C = O of enone).

EXAMPLE 25

N-[4-(2-Methoxyphenyl)piperazine-1-yl]ethyl-(3α,16α)-eburunamenine-14-caboxamide

To a stirred solution of (3α,16α)-eburunamenine-14-carbonyl chloride (2.04 g) in pyridine (35 ml) and benzene (20 ml) was added a solution of 1-(2-aminoethyl)-4-(2-methoxyphenyl)piperazine (1.61 g) in benzene (8 ml). The mixture was refluxed for 2 hours and concentrated in vacuo. To the residue was added water (100 ml) and the mixture was treated with 25%-ammonia water (pH 9 to 10), then extracted with chloroform (100 ml×2). The organic layer was washed successively with 0.05N-hydrochloric acid, water and brine, dried over sodium sulfate, and concentrated in vacuo. The residue was purified by column chromatography (Merck Silicagel 60) using chloroform/ethanol (50:1 to 10:1) as eluent to give 2.33 g (70.0%) of the title compound as yellowish crystals.
MS Spectrum m/z:
EI/DI; 539 ($M^+$), 205 (base).
$^1$H-NMR Spectrum ($CDCl_3$) δ ppm:
1.08-1.17 (1H, m, $CH_2$), 1.10 (3H, t, J = 7.3Hz, $CH_3$), 1.46-1.54 (1H, m, $CH_2$), 1.58-1.65 (1H, m, $CH_2$), 1.99 (2H, q, J = 7.3Hz, $CH_2$), 2.64-2.77 (8H, m, $CH_2$), 3.03-3.12 (6H, m, $CH_2$), 3.35-3.44 (2H, m, $CH_2$), 3.72 (2H, q, J = 5.9Hz, N-$CH_2$), 3.95 (3H, S, $OCH_3$), 4.27 (1H, s, $C_3$-H), 5.83 (1H, s, C = CH), 6.65 (1H, t, J = 4.9Hz, CONH), 6.93-7.10 (4H, m, ArH), 7.13-7.27 (2H, m, ArH), 7.38-7.43 (1H, m, ArH), 7.54-7.58 (1H, m, ArH).
IR Spectrum (KBr) ν $cm^{-1}$:
3287 (N-H), 2937, 2820 (C-H), 1658 (C = O), 1631, 1500, 1454 (C = C).

EXAMPLE 26

N-[4-(2-Chlorophenyl)piperazine-1-yl]ethyl-(3α,16α)-eburunamenine-14-caboxamide

Using a similar procedure to that described in Example 25 except that 1-(2-aminoethyl)-4-(2-chlorophenyl)piperazine (1.63 g) was used instead of 1-(2-aminoethyl)-4-(2-methoxyphenyl)piperazine, the title compound [2.43 g (72.1%)] was obtained as colorless powders.
MS Spectrum m/z:
EI/DI; 545, 543 ($M^+$), 209 (base).
$^1$H-NMR Spectrum ($CDCl_3$) δ ppm:
0.97-1.08 (1H, m, $CH_2$), 1.01 (3H, t, J = 7.3Hz, $CH_3$), 1.36-1.43 (1H, m, $CH_2$), 1.50-1.55 (1H, m, $CH_2$), 1.90 (2H, q, J = 7.3Hz, $CH_2$), 2.46-2.54 (1H, m, $CH_2$), 2.57-2.68 (8H, m, $CH_2$), 2.92-3.08 (6H, m, $CH_2$), 3.20-3.38 (2H, m, $CH_2$), 3.63 (2H, q, J = 5.9Hz, N-$CH_2$), 4.18 (1H, s, $C_3$-H), 5.75 (1H, s, C = CH), 6.52 (1H, t, J = 4.9Hz, CONH), 6.93-7.09 (2H, m, ArH), 7.11-7.15 (2H, m, ArH), 7.17-7.24 (1H, m, ArH), 7.29-7.36 (2H, m, ArH), 7.44-7.49 (1H, m, ArH).
IR Spectrum (KBr) ν $cm^{-1}$:
3283 (N-H), 2936, 2820 (C-H), 1658 (C = O), 1631, 1589, 1479, 1454 (C = C).

EXAMPLE 27

(3α,16α)-Eburunamenine-14-carboxylic acid 2-[4-(2-methoxyphenyl)piperazine-1-yl]ethyl ester

To a stirred solution of (3α,16α)-eburunamenine-14-carbonyl chloride (2.11 g) in pyridine (40 ml) and benzene (10 ml) was added a solution of 1-(2-hydroxyethyl)-4-(2-methoxyphenyl)piperazine (1.47 g) in benzene (10 ml). The mixture was refluxed for 2 hours and concentrated in vacuo. To the residue was added water (100 ml) and the mixture was treated with 25%-ammonia water (pH 9 to 10), then extracted with chloroform (100 ml×2). The organic layer was washed successively with 0.05N-hydrochloric acid, water and brine, dried over sodium sulfate, and concentrated in vacuo. The residue was purified by column

chromatography (Merck Silicagel Art. 7734) using chloroform/methanol methanol (50:1) as eluent to give 3.12 g (93.0%) of the title compound as yellowish crystals.
MS Spectrum m/z:
EI/DI; 540 ($M^+$, base).
$^1$H-NMR Spectrum ($CDCl_3$) $\delta$ ppm:
0.99-1.07 (1H, m, $CH_2$), 1.01 (3H, t, J = 7.3Hz, $CH_3$), 1.38-1.42 (1H, m, $CH_2$), 1.49-1.54 (1H, m, $CH_2$), 1.67-1.76 (1H, m, $CH_2$), 1.91 (2H, q, J = 7.3Hz, $CH_2$), 2.47-2.54 (1H, m, $CH_2$), 2.60-2.64 (2H, m, $CH_2$), 2.77-2.84 (6H, m, $CH_2$), 2.97-3.11 (5H, m, $CH_2$), 3.19-3.39 (2H, m, $CH_2$), 3.87 (3H, S, $OCH_3$), 4.14 (1H, s, $C_3$-H), 4.53 (2H, q, J = 5.9Hz, N-$CH_2$), 6.15 (1H, s, C = CH), 6.85-7.04 (4H, m, ArH), 7.08-7.18 (2H, m, ArH), 7.35-7.38 (1H, m, ArH), 7.44-7.47 (1H, m, ArH).

## EXAMPLE 28

(3α,16α)-Eburunamenine-14-carboxylic acid 2-[4-(2-chlorophenyl)piperazine-1-yl]ethyl ester

Using a similar procedure to that described in Example 27 except that 1-(2-hydroxyethyl)-4-(2-chlorophenyl)piperazine (1.50 g) was used instead of 1-(2-hydroxyethyl)-4-(2-methoxyphenyl)piperazine, the title compound [2.88 g (85.2%)] was obtained as colorless powders.
MS Spectrum m/z:
EI/DI; 546, 544 ($M^+$, base).
$^1$H-NMR Spectrum ($CDCl_3$) $\delta$ ppm:
0.97-1.08 (1H, m, $CH_2$), 1.02 (3H, t, J = 7.3Hz, $CH_3$), 1.36-1.44 (1H, m, $CH_2$), 1.49-1.54 (1H, m, $CH_2$), 1.67-1.76 (1H, m, $CH_2$), 1.91 (2H, q, J = 7.3Hz, $CH_2$), 2.46-2.54 (1H, m, $CH_2$), 2.60-2.65 (2H, m, $CH_2$), 2.69-2.88 (6H, m, $CH_2$), 2.96-3.11 (5H, m, $CH_2$), 3.19-3.39 (2H, m, $CH_2$), 4.15 (1H, s, $C_3$-H), 4.53 (2H, q, J = 5.9Hz, N-$CH_2$), 6.15 (1H, s, C = CH), 6.93-7.06 (2H, m, ArH), 7.08-7.30 (4H, m, ArH), 7.34-7.39 (1H, m, ArH), 7.44-7.48 (1H, m, ArH).

## TEST EXAMPLE 1
(Pharmacological example)

1. Antiviral activities against herpes simplex virus type 1 (HSV-1) in vitro and cytotoxicity:

Confluent GMK cells (derived from green monkey kidney) in 96-well plates were infected with HSV-1 (Miyama strain) in the presence of various concentrations of test compounds. After incubation, virus-induced cytopathic effect (CPE), inhibitory effect of CPE by the test compound and cytotoxicity were microscopically observed. The virus titer ($TCID_{50}$) was determined from CPE of virus infected culture. Antivirus effects of test compounds were calculated from $TCID_{50}$ values of test compound treated and control cultures and represented by $\Delta TCID_{50}(\log_{10})$. Test compounds were dissolved in MEM medium, ethanol or DMSO to 10 mg/ml, and diluted with MEM medium supplemented with 1% fetal bovineserum.

TABLE 1

| Antiviral activities of test compounds against herpes simplex virus type 1 (Miyama strain) in vitro | | |
|---|---|---|
| Test Compound | Antiviral Activity ($\Delta TCID_{50}(log_{10})$) | |
| | 5 µg/ml | 10 µg/ml |
| Compound of Example 1 | | 0.17 (-) |
| Compound of Example 2 | | 0.50 (-) |
| Compound of Example 3 | | 0.83 (-) |
| Compound of Example 4 | | 0.66 (-) |
| Compound of Example 5 | >2.83 (-) | >2.83 (±) |
| Compound of Example 6 | | 0.17 (-) |
| Compound of Example 7 | | 0.17 (-) |
| Compound of Example 8 | | 2.00 (-) |
| Compound of Example 9 | 0.83 (-) | >3.00 (±) |
| Compound of Example 10 | | 0.17 (-) |
| Compound of Example 11 | | 1.50 (-) |
| Compound of Example 12 | | 0.33 (-) |
| Compound of Example 13 | | 0.00 (-) |
| Compound of Example 14 | | 0.00 (-) |
| Compound of Example 15 | | >2.00 (-) |

In Table 1, parentheses indicate the cytotoxicity of these compounds. (-) and (±) indicate no cytotoxicity and slight cytotoxicity, respectively.

2. Antiviral activities of test compounds:

Monolayer cells in 96-well plates were infected with HSV-1 (KOS strain), HSV-2 (UW-268 strain), vaccinia virus (DIE strain) or influenza virus (A/PR/8 strain) in the presence of test compound. After incubation, virus-induced cytopathic effect (CPE), inhibitory effect of CPE by the test compound and cytotoxicity were microscopically observed. The virus titer ($TCID_{50}$) was determined from CPE of virus infected culture. Antivirus effects of test compounds were calculated from $TCID_{50}$ values of compound treated and control cultures. Results were represented by $\Delta TCID_{50}(log_{10})$. The host cells were MDCK cells for influenza virus and Vero cells for other viruses.

Test compounds were dissolved in MEM medium to 10 mg/ml, and diluted with MEM medium supplemented with 1% fetal bovine serum.

TABLE 2

Antiviral activities of test compounds

| Virus | Antiviral Activity ($\Delta TCID_{50}(log_{10})$) 5 μg/ml | 10 μg/ml | 50 μg/ml |
|---|---|---|---|
| Compound of Example 5: | | | |
| HSV-1 | 3.00 (-) | >3.50 (-) | >4.00 (±) |
| HSV-2 | 2.00 (-) | >3.00 (-) | >3.66 (±) |
| Vaccinia virus | 1.00 (-) | >2.83 (-) | >3.50 (±) |
| Influenza virus | 2.00 (-) | >2.50 (-) | |
| Compound of Example 8: | | | |
| HSV-1 | | 1.67 (-) | 2.00 (-) |
| HSV-2 | | 1.00 (-) | 2.00 (-) |
| Vaccinia virus | 0.88 (-) | 2.00 (-) | 2.50 (-) |
| Influenza virus | 1.00 (-) | 2.00 (-) | 2.00 (-) |
| Compound of Example 9: | | | |
| HSV-1 | 2.00 (-) | >3.50 (±) | |
| HSV-2 | 1.50 (-) | >4.00 (±) | |
| Vaccinia virus | 1.67 (-) | >3.00 (±) | |
| Influenza virus | 1.50 (-) | >3.00 (±) | |
| Compound of Example 11: | | | |
| HSV-1 | 0.83 (-) | 1.17 (-) | 3.50 (+) |
| HSV-2 | | 1.00 (-) | 2.50 (+) |
| Vaccinia virus | | 1.00 (-) | 2.00 (+) |
| Influenza virus | | 1.83 (-) | 3.00 (+) |

In Table 2, parentheses indicate the cytotoxicity of these compounds. (-), (±) and (+) indicate no cytotoxicity, slight cytotoxicity and cytotoxicity with observable antiviral effect, respectively.

3. Anti HSV-1 activities of test compound in vivo:

Male BALB/c mice (5 weeks of age) were intraperitoneally infected with 322 PFU (plaque forming unit, $10LD_{50}$) of HSV-1 (Miyama strain). Twenty one days later, survival ratio and mean survival days were evaluated. Test compounds were dispersed in 5% arabic gum/physiological saline, and intraperitoneally administered at 10 mg and 20 mg/kg. Treatment began just after virus inoculation and continued for 5 days.

The results were shown in Table 3. Significant increase of survival ratio and mean survival days were observed in the compound treated group.

TABLE 3

Anti HSV-1 activities of test compound in vivo

| Dose | Survival Ratio at 21 days (%) | Mean Survival Days (day) |
|---|---|---|
| Compound of Example 5 | | |
| 0 mg/kg | 0 | 6.6±0.5 |
| 10 mg/kg | 40 | 16.8±1.6 *** |
| 20 mg/kg | 70 | 18.7±1.2 *** |
| Compound of Example 8 | | |
| 0 mg/kg | 0 | 6.6±0.5 |
| 10 mg/kg | 20 | 13.7±1.6 *** |
| 20 mg/kg | 40 | 16.4±2.0 *** |
| Compound of Example 9 | | |
| 0 mg/kg | 0 | 6.6±0.5 |
| 10 mg/kg | 20 | 14.2±1.5 *** |
| 20 mg/kg | 60 | 18.7±1.8 *** |

***: Significant difference from 0 mg/kg group using U Test.

TEST EXAMPLE 2
(Pharmacological example)

1. Antiviral activities against herpes simplex virus type 1 (HSV-1) in vitro and cytotoxicity:

Confluent GMK cells (derived from green monkey kidney) in 96-well plates were infected with HSV-1 (Miyama strain) in the presence of various concentrations of test compounds. After incubation, virus-induced cytopathic effect (CPE), inhibitory effect of CPE by the test compound and cytotoxicity were microscopically observed. The virus titer ($TCID_{50}$) was determined from CPE of virus infected culture. Ancivirus effects of test compounds were calculated from $TCID_{50}$ values of test compound treated and control cultures and represented by $\Delta TCID_{50}(\log_{10})$. Test compounds were dissolved in MEM medium, ethanol or DMSO to 10 mg/ml, and diluted with MEM medium supplemented with 0.5% fetal bovine serum.

TABLE 4

| Antiviral activities of test compounds against herpes simplex virus type 1 (Miyama strain) in vitro | | |
|---|---|---|
| Test Compound | Antiviral Activity ($\Delta TCID_{50}(log_{10})$) | |
| | 5 µg/ml | 10 µg/ml |
| Compound of Example 16 | 2.16 (-) | >3.00 (+) |
| Compound of Example 17 | | 1.56 (-) |
| Compound of Example 18 | | 1.67 (-) |
| Compound of Example 19 | 1.17 (-) | >3.00 (+) |
| Compound of Example 20 | 1.66 (-) | >2.83 (±) |
| Compound of Example 21 | | 1.00 (-) |
| Compound of Example 22 | | 0.64 (-) |
| Compound of Example 23 | >3.17 (±) | >3.17 (+) |
| Compound of Example 24 | | 0.33 (-) |
| Compound of Example 25 | 0.67 (-) | 2.27 (-) |
| Compound of Example 26 | 2.00 (-) | >3.17 (+) |
| Compound of Example 27 | 1.00 (-) | 3.00 (±) |
| Compound of Example 28 | 2.00 (-) | 3.00 (+) |

In Table 4, parentheses indicate the cytotoxicity of these compounds. (-), (±) and (+) indicate no cytotoxicity, slight cytotoxicity and cytotoxicity with observable antiviral effect, respectively.

2. Antiviral activities of test compounds:

Monolayer cells in 96-well plates were infected with HSV-1 (KOS strain), HSV-2 (UW-268 strain), vaccinia virus (DIE strain) or influenza virus (A/PR/8 strain) in the presence of test compound. After incubation, virus-induced cytopathic effect (CPE), inhibitory effect of CPE by the test compound and cytotoxicity were microscopically observed. The virus titer ($TCID_{50}$) was determined from CPE of virus infected culture. Antivirus effects of test compounds were calculated from $TCID_{50}$ values of compound treated and control cultures. Results were represented by $\Delta TCID_{50}(log_{10})$. The host cells were MDCK cells for influenza virus and Vero cells for other viruses.

Test compounds were dissolved in MEM medium to 10 mg/ml, and diluted with MEM medium supplemented with 1% fetal bovine serum.

TABLE 5

Antiviral activities of test compounds

| Virus | Antiviral Activity ($\Delta TCID_{50}(\log_{10})$) 5 μg/ml | 10 μg/ml |
|---|---|---|
| Compound of Example 16 | | |
| HSV-1 | 2.16 (-) | >3.00 (+) |
| HSV-2 | 2.00 (-) | >3.00 (+) |
| Vaccinia virus | 1.66 (-) | 3.00 (+) |
| Influenza virus | 2.00 (-) | >2.83 (±) |
| Compound of Example 23 | | |
| HSV-1 | >3.50 (+) | >3.50 (+) |
| HSV-2 | >3.00 (+) | >3.00 (+) |
| Vaccinia virus | 2.56 (+) | >3.50 (+) |
| Influenza virus | 2.00 (-) | 2.80 (+) |
| Compound of Example 26 | | |
| HSV-1 | 1.83 (-) | >3.00 (+) |
| HSV-2 | 1.65 (-) | 3.00 (+) |
| Vaccinia virus | 1.50 (-) | 3.00 (+) |
| Influenza virus | 1.55 (-) | 3.50 (±) |

In Table 5, parentheses indicate the cytotoxicity of these compounds. (-), (±) and (+) indicate no cytotoxicity, slight cytotoxicity and cytotoxicity with observable antiviral effect, respectively.

3. Inhibitory effects of test compounds on HIV-induced cytopathogenicity:

MT-4 cells (lymphoma derived from human T cell leukemia) in 96-well plate were infected with HSV-1 in the presence of various concentrations of test compounds. After incubation, inhibitory effect of HIV-induced CPE by the test compounds and cytotoxicity were microscopically observed. Test compounds protected the cells against virus-induced cell destraction.

TABLE 6

| Inhibitory effects of test compounds on HIV-induced cytopathogenicity | |
|---|---|
| Test Compounds | Anti-HIV Activity (MIC, µg/ml) |
| Compound of Example 8 | 16 (>125) |
| Compound of Example 9 | >125 (>250) |
| Compound of Example 16 | > 63 (125) |
| Compound of Example 19 | 31 (>125) |
| Compound of Example 27 | 8 (63) |
| Compound of Example 28 | 4 (63) |

In Table 6, parentheses indicate the minimum cytotoxic concentration of test compounds.

MIC: minimum inhibitory concentration

The phenylpiperazine derivatives, phenylpiperidine derivatives, phenylpyrrolidine derivatives, phenyltetrahydroimidazole derivatives, phenylhomopiperazine derivatives, phenylaziridine derivatives, phenylazetidine derivatives, phenyldiazetidine derivatives, and phenylperhydroazepine derivatives according to the present invention possess excellent antiviral activities, and are useful for preventing or treating infectious diseases caused by various DNA viruses, RNA viruses, and retro-viruses. The compounds of the present invention possess a wide and potent antiviral activity spectrum as compared with the conventional antiviral substances. Further, since the compounds of the present invention have a chemical structure different from the conventional antiviral substances, it is useful for the prevention or treatment of the infectious diseases caused by viruses resistant to the conventional antiviral substances.

While the invention has been described in detail and with reference to specific embodiments thereof, it will be apparent to one skilled in the art that various changes and modifications can be made therein without departing from the spirit and scope thereof.

**Claims**

1. An antiviral agent containing a compound represented by formula (I):

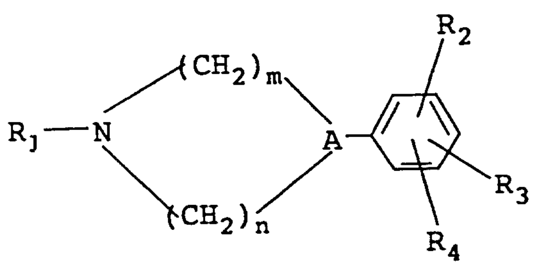

(I)

wherein $R_1$ represents a substituted or unsubstituted alkyl group, a substituted or unsubstituted acyl group, a substituted or unsubstituted arylsulfonyl group, a substituted or unsubstituted alkylsulfonyl group, a substituted or unsubstituted phenyl group, or a substituted or unsubstituted heterocyclic group; $R_2$, $R_3$, and $R_4$, which may be the same or different, each represent a hydrogen atom, an amino group, a substituted or unsubstituted alkylamino group, an acylamino group, a substituted or unsubstituted alkyl group, a hydroxyl group, a substituted or unsubstituted alkyloxy group, a halogen atom, a carboxyl group, a substituted or unsubstituted alkylcarbonyl group, a substituted or unsubstituted alkoxycarbonyl group, a substituted or unsubstituted aryloxycarbonyl group, a substituted or unsubstituted carbamoyl group, a nitro group, a cyano group, a thiol group, a substituted or unsubstituted alkylthio group, a substituted or unsubstituted phenyl group, or a substituted or unsubstituted heterocyclic group; A represents a nitrogen group or a methylene group; m represents 0 or a natural number; and n represents a natural number, or a pharmaceutically acceptable salt thereof.

2. An antiviral agent as claimed in Claim 1, wherein said compound represented by formula (I) is selected from 1-acetyl-4-phenylpiperazine, 1-acetyl-4-(o-, m- or p-chlorophenyl)piperazine, 1-acetyl-4-(o-, m- or p-trifluoromethylphenyl)piperazine, 1-phenyl-4-decanoylpiperazine, 1-(o-, m- or p-chlorophenyl)-4-decanoylpiperazine, 1-(o-, m- or p-trifluoromethylphenyl)-4-decanoylpiperazine, 1-octanoyl-4-phenylpiperazine, 1-(o-, m- or p-chlorophenyl)-4-octanoylpiperazine, 1-(o-, m- or p-trifluoromethylphenyl)-4-octanoylpiperazine, 1-phenyl-4-octadecanoylpiperazine, 1-(o-, m- or p-chlorophenyl)-4-octadecanoylpiperazine, 1-(o-, m- or p-trifluoromethylphenyl)-4-octadecanoylpiperazine, 1-[(Z,Z)-9,12-octadecadienoyl]-4-phenylpiperazine, 1-(o-, m- or p-chlorophenyl)-4-[(Z,Z)-9,12-octadecadienoyl]-piperazine, 1-(o-, m- or p-trifluoromethylphenyl)-4-[(Z,Z)-9,12-octadecadienoyl]piperazine, 1-(5β-cholan-24-oyl)-4-phenylpiperazine, 1-(o-, m- or p-chlorophenyl)-4-(5β-cholan-24-oyl)piperazine, 1-(5β-cholan-24-oil)-4-(o-, m- or p-trifluoromethylphenyl)piperazine, 1-(4-aminophenylsulfonyl)-4-phenylpiperazine, 1-(4-aminophenylsulfonyl)-4-(o-, m- or p-chlorophenyl)piperazine, 1-(4-aminophenylsulfonyl)-4-(o-, m- or p-trifluoromethylphenyl)piperazine, 1-ethyl-4-phenylpiperazine, 1-(o-, m- or p-chlorophenyl)-4-ethylpiperazine, 1-ethyl-4-(o-, m- or p-trifluoromethylphenyl)piperazine, 1-phenyl-4-(2-pyrrolidon-1-yl)-acetylpiperazine, 1-(o-, m- or p-chlorophenyl)-4-(2-pyrrolidon-1-yl)acetylpiperazine, 1-(o-, m- or p-trifluoromethylphenyl)-4-(2-pyrrolidon-1-yl)acetylpiperazine, 1-(2-acetoxybenzoyl)-4-phenylpiperazine, 1-(2-acetoxybenzoyl)-4-(o-, m- or p-chlorophenyl)piperazine, 1-(2-acetoxybenzoyl)-4-(o-, m- or p-trifluoromethylphenyl)piperazine, 1-(2-hydroxybenzoyl)-4-phenylpiperazine, 1-(2-hydroxybenzoyl)-4-(o-, m- or p-chlorophenyl)piperazine, 1-(2-hydroxybenzoyl)-4-(o-, m- or p-trifluoromethylphenyl)piperazine, 1-(1-azabicyclo[3.3.0]octan-5-yl)-4-phenylpiperazine, 1-(1-azabicyclo[3.3.0]octan-5-yl)-4-(o-, m- or p-chlorophenyl)piperazine, 1-(1-azabicyclo[3.3.0]octan-5-yl)-4-(o-, m- or p-trifluoromethylphenyl)-piperazine, L-glutamic acid α-(4-phenylpiperazine)amide, L-glutamic acid α-[4-(o-, m- or p-chlorophenyl)piperazine]amide, L-glutamic acid α-[4-(o-, m-or p-trifluoromethylphenyl)piperazine]amide, 1-(3β-hydroxy-18β-olean-12-en-30-oyl)-4-phenylpiperazine, 1-(3β-hydroxy-18β-olean-12-en-30-oyl)-4-(o-, m- or p-chlorophenyl)piperazine, 1-(o-, m- or p-trifluoromethylphenyl)-4-(3β-hydroxy-18β-olean-12-en-30-oyl)piperazine, and pharmaceutically acceptable salts thereof.

**3.** An antiviral agent as claimed in Claim 1, wherein said antiviral agent is a prophylactic or treating agent for infectious diseases caused by DNA viruses, RNA viruses or retroviruses.

**4.** An antiviral agent as claimed in Claim 1, wherein said antiviral agent is a treating agent for infectious diseases caused by viruses belonging to Herpesviridae.

**5.** An antiviral agent as claimed in Claim 1, wherein said antiviral agent is a treating agent for influenza virus infectious diseases.

**6.** An easily absorbable pharmaceutical composition comprising an antiviral agent containing a compound represented by formula (I):

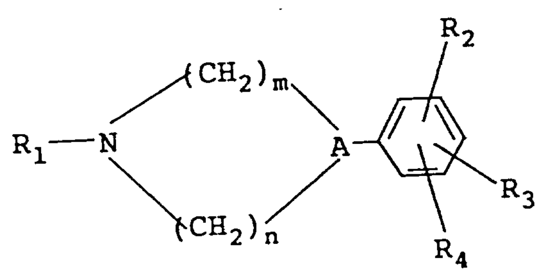

(I)

wherein $R_1$ represents a substituted or unsubstituted alkyl group, a substituted or unsubstituted acyl group, a substituted or unsubstituted arylsulfonyl group, a substituted or unsubstituted alkylsulfonyl group, a substituted or unsubstituted phenyl group, or a substituted or unsubstituted heterocyclic group; $R_2$, $R_3$, and $R_4$, which may be the same or different, each represent a hydrogen atom, an amino group, a substituted or unsubstituted alkylamino group, an acylamino group, a substituted or unsubstituted alkyl group, a hydroxyl group, a substituted or unsubstituted alkyloxy group, a halogen atom, a carboxyl group, a substituted or unsubstituted alkylcarbonyl group, a substituted or unsubstituted alkoxycarbonyl group, a substituted or unsubstituted aryloxycarbonyl group, a substituted or unsubstituted carbamoyl group, a nitro group, a cyano group, a thiol group, a substituted or unsubstituted alkylthio group, a substituted or unsubstituted phenyl group, or a substituted or unsubstituted heterocyclic group; A represents a nitrogen group or a methylene group; m represents 0 or a natural number; and n represents a natural number,

or a pharmaceutically acceptable salt thereof, and a polyoxyethylene higher alcohol ether or a surface active agent.

European Patent Office

# PARTIAL EUROPEAN SEARCH REPORT

which under Rule 45 of the European Patent Convention shall be considered, for the purposes of subsequent proceedings, as the European search report

Application Number

EP 92 12 1466

## DOCUMENTS CONSIDERED TO BE RELEVANT

| Category | Citation of document with indication, where appropriate, of relevant passages | Relevant to claim |
|---|---|---|
| X,P | EP-A-0 496 222 (MERCK PATENT GMBH)<br>* examples *<br>--- | 1 |
| X | EP-A-0 458 618 (ORTHO PHARMACEUTICAL CORPORATION)<br>* example 10 *<br>--- | 1 |
| X | EP-A-0 457 140 (BAYER A.G.)<br>* example 26 *<br>--- | 1 |
| X | EP-A-0 395 093 (KYOWA HAKKO KOGYO CO. LTD.)<br>* table 1 *<br>--- | 1 |
| X | EP-A-0 301 549 (FERRER INTERNACIONAL S.A.)<br>* examples *<br>--- -/- | 1 |

CLASSIFICATION OF THE APPLICATION (Int. Cl.5)

C 07 D 295/18
C 07 D 295/22
C 07 D 295/06
C 07 D 403/06
C 07 D 401/04
C 07 D 403/04
C 07 D 405/12
C 07 D 471/04
C 07 D 473/30
A 61 K 31/495

TECHNICAL FIELDS SEARCHED (Int. Cl.5)

C 07 D
A 61 K
C 07 C
A 01 N

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely : 2 - 6

Claims searched incompletely : 1

Claims not searched :

Reason for the limitation of the search:

| Place of search | Date of completion of the search | Examiner |
|---|---|---|
| BERLIN | 10-03-1993 | FRELON D L M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

European Patent Office

# PARTIAL EUROPEAN SEARCH REPORT

Application Number

EP 92 12 1466

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 241 053 (DUPHAR INTERNATIONAL RESEARCH B.V.)<br>* pages 10-14; examples *<br>--- | 1 | |
| X | EP-A-0 175 541 (WARNER-LAMBERT CO.)<br>* examples 1-4 *<br>--- | 1 | |
| X | EP-A-0 173 331 (OTSUKA PHARMACEUTICAL CO. LTD.)<br>* pages 110-123,128-130 *<br>--- | 1 | |
| X | EP-A-0 171 636 (RAVIZZA S.P.A.)<br>* examples 13-15 *<br>--- | 1 | |
| X | EP-A-0 150 036 (HOECHST A.G.)<br>* table 1, pages 52-61; examples 10,16 *<br>--- | 1 | TECHNICAL FIELDS SEARCHED (Int. Cl.5) |
| X | EP-A-0 138 720 (DROPIC SOC. CIVILLE DE GESTION DE DROIT DE PROPRIETE INDUSTRIELLE CHOAY)<br>* page 57; examples *<br>--- | 1 | |
| X | EP-A-0 120 465 (DEGUSSA A.G.)<br>* pages 23-39; examples *<br>--- | 1 | |
| X | EP-A-0 117 531 (BOEHRINGER INGELHEIM K.G.)<br>* examples *<br>--- | 1 | |
| X | EP-A-0 109 562 (SUMITOMO CHEMICAL CO. LTD.)<br>* ref. examples 6,8 *<br>--- | 1 | |
| X | EP-A-0 094 498 (AMERICAN CYANAMID CO.)<br>* examples 18,19 *<br>--- | 1 | |
| X | EP-A-0 076 996 (BOEHRINGER MANNHEIM GMBH.)<br>* examples 5-13 *<br>--- -/- | 1 | |

European Patent Office

# PARTIAL EUROPEAN SEARCH REPORT

Application Number

EP 92 12 1466

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.5) |
|---|---|---|---|
| X | EP-A-0 050 072 (INNOTHERA)<br>* examples 10-12 *<br>--- | 1 | |
| X | EP-A-0 007 482 (CELAMERCK GMBH & CO. KG.)<br>* example 75 *<br>--- | 1 | |
| X | EP-A-0 006 722 (JANSSEN PHARMACEUTICA N.V.)<br>* examples II,III *<br>--- | 1 | |
| X | EP-A-0 005 142 (F. HOFFMANN-LA ROCHE & CO.)<br>* examples *<br>--- | 1 | TECHNICAL FIELDS SEARCHED (Int. Cl.5) |
| X | WO-A-9 118 597 (R. ERICKSON et al.)<br>* example 63 *<br>--- | 1 | |
| X | WO-A-9 109 849 (THE UPJOHN CO.)<br>* pages 62,63,68,79 *<br>--- | 1 | |
| X | WO-A-9 109 594 (VIRGINIA COMMONWEALTH UNIVERSITY)<br>* pages 43,52,53,55; examples *<br>--- | 1 | |
| X | WO-A-9 008 762 (IMPERIAL CHEMICAL INDUSTRIES PLC.)<br>* examples 5,11 *<br>--- | 1 | |
| X | SYNTHESIS no. 12, 1990, pages 1145-1147, Stuttgart, DE; H. KOTSUKI et al.: "High Pressure Organic Chemistry; XI. A New Convenient Synthesis of Aromatic Amines from Activated Phenols"<br>* examples 3b,d *<br>--- | 1 | |
| X | SYNTHESIS no. 12, 1990, page 1147, Stuttgart, DE; H. KOTSUKI et al.: "High Pressure Organic Chemistry; XII. A Convenient Synthesis of Aromatic Amines from Activated Aromatic Fluorides"<br>* examples 2e,g,n *<br>--- -/- | 1 | |

European Patent Office

# PARTIAL EUROPEAN SEARCH REPORT

Application Number
EP 92 12 1466

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 576 021 (CENTRE NATIONAL DE LA RECHERCHE SCIENTIFIQUE)<br>* pages 10-11; tables 1,2 *<br>--- | 1 | |
| X | FR-A-2 533 564 (SELVI & C. S.P.A.)<br>* pages 16-17; table II *<br>--- | 1 | |
| X | FR-A-2 492 825 (NIPPON KAYAKU KABUSHIKI KAISHA)<br>* pages 5-11 *<br>--- | 1 | |
| X | FR-A-2 373 537 (BEECHAM GROUP LTD.)<br>* examples 2,4,5 *<br>--- | 1 | |
| X | FR-A-2 372 827 (ABBOTT LABORATORIES)<br>* example 8 *<br>--- | 1 | TECHNICAL FIELDS SEARCHED (Int. Cl.5) |
| X | FR-A-2 139 158 (AMERICAN CYANAMID CO.)<br>* pages 31-48; examples *<br>--- | 1 | |
| X | FR-M- 6 361 (A.H. ROBINS CO., INC.)<br>* example 10 *<br>--- | 1 | |
| X | CH-A- 634 298 (CIBA-GEIGY A.G.)<br>* examples *<br>--- | 1 | |
| X | CH-A- 634 299 (CIBA-GEIGY A.G.)<br>* examples *<br>--- | 1 | |
| X | US-A-4 857 644 (M.A. ABOU-GHARBIA.)<br>* example 3 *<br>--- | 1 | |
| X | US-A-4 397 855 (I. SIRCAR)<br>* table; columns 5,6 *<br>--- -/- | 1 | |

European Patent Office

# PARTIAL EUROPEAN SEARCH REPORT

Application Number
EP 92 12 1466

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| X | US-A-4 159 331 (J.M. MCCALL)<br>* examples *<br>--- | 1 | |
| X | US-A-3 988 457 (H.F. HILL et al.)<br>* examples 4-7 *<br>--- | 1 | |
| X | US-A-3 946 007 (C.T. GORALSKI et al.)<br>* table I, c) *<br>--- | 1 | |
| X | GB-A-2 078 746 (TAIHO PHARMACEUTICAL CO., LTD.)<br>* examples *<br>--- | 1 | |
| X | DE-A-3 238 007 (BAYER A.G.)<br>* pages 10,20-24,26; examples *<br>--- | 1 | TECHNICAL FIELDS SEARCHED (Int. Cl.5) |
| X | DE-A-2 914 491 (THE UPJOHN CO.)<br>* examples 1-3,5-8,21-24; pages 54,56 *<br>--- | 1 | |
| X | DE-A-2 804 096 (JANSSEN PHARMACEUTICA N.V.)<br>* pages 29-34,44-50,54-68; examples *<br>--- | 1 | |
| X | DE-A-2 753 878 (BEECHAM GROUP LTD.)<br>* examples *<br>--- | 1 | |
| X | DE-A-2 328 053 (E. BOUCHARA)<br>* examples *<br>--- | 1 | |
| A | DATABASE WPIL Derwent Publications Ltd., London, GB; DATABASE WPIL, accession no. 77-65668y, week 7737; & JP - A - 52091883 (MORISHITA SEIYAKU K.)<br>* abstract *<br>--- -/- | 1-6 | |

European Patent Office

# PARTIAL EUROPEAN SEARCH REPORT

Application Number
EP 92 12 1466

| Category | Citation of document with indication, where appropriate, of relevant passages | Relevant to claim | CLASSIFICATION OF THE APPLICATION (Int. Cl.5) |
|---|---|---|---|
| A,P | EP-A-0 518 533 (SANWA KAGAKU KENKYUSHO)<br>* groups II,III,IV * | 1-6 | |
| D,X | EP-A-0 156 433 (JANSSEN PHARMACEUTICA N.V.)<br>* pages 30-35,37,40,42,44-46,49,50,52,53,56-60; examples * | 1 | |
| A | * the whole document * | 1-6 | |
| A,D | ANTIMICROBIAL AGENTS AND CHEMOTHERAPY vol. 30, no. 2, August 1986, pages 215-219; J.C. POTTAGE et al.: "In Vitro Activity of Ketoconazole against Herpes Simplex Virus" | 1-6 | TECHNICAL FIELDS SEARCHED (Int. Cl.5) |
| A | EP-A-0 398 426 (JANSSEN PHARMACEUTICA N.V.)<br>* abstract * | 1-6 | |